# EUROPEAN PATENT APPLICATION

(11) **EP 2 813 176 A2**
(43) Date of publication of application: **17.12.2014**
(21) Application number: 14171792.6
(22) Date of filing: 10.06.2014
(51) Int. Cl.: A61B 5/00, A61B 5/08, A61B 5/113

(54) **Optical respiration sensor**

(30) Priority: 10.06.2013 US 201361833062 P
(71) Applicant: Digisense Ltd., 49220 Petach Tikva (IL); Expro3, LLC, Coral Gables, FL 33134 (US)
(72) Inventor: Abir, Eyall, Petach Tikva (IL)
(74) Representative: Crease, Devanand John

(57) **Abstract**

A garment-mountable respiration sensor, comprising: a light source configured to illuminate said garment; a photodetector configured to sense an amount of light reflected from said garment; and an electronic circuit configured to detect respiration based on a temporal pattern of the amount of light sensed by said photodetector.

## Description

### FIELD OF THE INVENTION

The invention relates to the field of respiration sensors.

### BACKGROUND

A person's vital signs such as heart rate, temperature, respiration, or the like, may be monitored for a number of reasons. For instance, the vital signs of a patient in a hospital are monitored to provide information about the health status of the patient. Emergency responders monitor the vital signs of victims in the field to ascertain health status and perform triage. Athletes monitor their vital signs to improve training techniques and evaluate fitness levels. In short, vital signs may be monitored for any number of reasons. Typically, continual vital sign monitoring requires either hands-on human attention or bulky and expensive equipment. Although much of the equipment used in the medical and emergency professions is bulky and unwieldy, miniaturization of vital sign monitors is occurring, as technology allowed such systems to be simple and not expensive. The use of heart rate monitors by athletes is a prime example of such miniaturization. Heart rate monitoring devices, such as electronic sensing chest straps, communicate with a wristwatch or other device to display and record the heart rate of the athlete over a period of time.

For babies and infants, there is even a greater need to monitor their vital signs, since they are sometimes unable to express distress. Respiration monitoring has a significant importance, due to the SIDS (Sudden Infant Death Syndrome) phenomena.

The measurement of human respiration over time is usually effected at one of two distinct places. One consists of the measurement of the current of air in or near the nasal and oral cavity. The other method consists of the measurement of the change in position of the chest or the abdomen, that is, rise and fall due to breathing in and out.

International Patent Application Publication No. 2009083980 to Bar Hayim discloses a disposable monitor that includes an adhesive sensing unit that includes a flexible conductive wire wherein the sensing unit is attached to the skin of a person being monitored.

International Patent Application Publication No. 2009050702 to Rahamim discloses an apnea detector. It incorporates a capacitive type sensor that emits an alert signal upon detecting symptoms of apnea. In one embodiment, a detector unit is in communication with a curvature sensor adapted to detect a variable curvature of a subject body surface resulting from breathing patterns of a subject. The detector unit is attached to an article of clothing of the subject. A monitoring system comprises a detector unit for detecting one or more subject related parameters of interest and for emitting acoustical information after determining that a subject related parameter of interest has a predetermined status, and a stationary unit disposed within an audible range of the detector unit for receiving the emitted acoustical information.

U.S. Patent No. 5,515,865 to Scanlon discloses an apparatus to monitor a living being's breathing movement and, in the absence of breathing movement, the apparatus attempts to stimulate breathing. The apparatus has a base member that may be a pad placed under the baby's body.

U.S Patent No. 7,715,897 to Coulston discloses a garment and system including a monitoring fabric that exhibits a light reflection property and substantially no light transmission property when the fabric is illuminated with light having wavelength(s) in the range of 400 to 2200 nanometers. The amount of useful light reflected by the fabric into an aperture of acceptance defined with respect to an axis extending from the fabric relative to the amount of light lost to the aperture of acceptance detectably changes when the fabric stretches in response to motion, as the motion induced by physiological activity (e.g. heart rate). The system includes at least one radiation source and at least one radiation detector, with the detector disposed in the aperture of acceptance. The source and detector may be attached to the fabric in relative positions such that the reception of incident radiation by the detector is directly affected by a change in the amount of useful light reflected by the fabric into the aperture of acceptance as the fabric stretches in response to motion.

International Patent Application Publication No. 2007069111 to Goris et al. discloses a device for assessing the physical condition of a person comprises a belt to be arranged around the person's chest, a sensor unit for detecting a distance between ends of the belt, and a processor unit for processing output provided by the sensor unit during operation of the device. The sensor unit comprises a LED for emitting light, a phototransistor for generating an output signal on the basis of a received amount of light, and a light blocking element having a wedge-shaped light-varying section. The LED and the phototransistor are associated with one end of the belt, while the light-blocking element is associated with the other end of the belt. As a result, when the person inhales and exhales, the amount of light that is received by the phototransistor varies, and an output signal of the phototransistor may be used as a measure of the chest circumference.

The foregoing examples of the related art and limitations related therewith are intended to be illustrative and not exclusive. Other limitations of the related art will become apparent to those of skill in the art upon a reading of the specification and a study of the figures.

### SUMMARY

The following embodiments and aspects thereof are described and illustrated in conjunction with systems, tools and methods which are meant to be exemplary and illustrative, not limiting in scope.

There is provided, in accordance with an embodiment, a garment-mountable respiration sensor, comprising: a light source configured to illuminate said garment; a photodetector configured to sense an amount of light reflected from said garment; and an electronic circuit configured to detect respiration based on a temporal pattern of the amount of light sensed by said photodetector.

There is further provided, in accordance with an embodiment, a respiration monitoring system comprising: a garment-mountable respiration sensor comprising:
(a) a light source configured to illuminate said garment, (b) a photodetector configured to sense an amount of light reflected from said garment, (c) a transducing element, (d) an electronic circuit configured to detect respiration based on a temporal pattern of the amount of light sensed by said photodetector, wherein said electronic circuit further comprises an audio encoder configured to encode a signal related to the detected respiration using said transducing element, and to cause said transducing element to transmit the encoded signal as an acoustic signal; and a receiving device configured to receive and decode the acoustic signal, and to convey the signal related to the detected respiration to a user.

In some embodiments, said light source is a white LED (Light Emitting Diode).

In some embodiments, said light source is a red LED (Light Emitting Diode).

In some embodiments, said sensor comprises a phototransistor configured to output a signal in relation to the amount of light sensed.

In some embodiments, said phototransistor is configured to sense light in the visible light range.

In some embodiments, said phototransistor is configured to sense light in the IR (Infra Red) range.

In some embodiments, said electronic circuit comprises a filter configured to filter an output voltage of said photodetector.

In some embodiments, said electronic circuit comprises a logic gate configured to convert an analog output voltage of said photodetector to digital data, and output an alert in case of deviation of said output voltage frequency from a pre-determined frequency.

In some embodiments, said electronic circuit comprises a threshold comparator configured to convert an analog output voltage of said photodetector to digital data, and output an alert in case of deviation of said output voltage frequency from a pre-determined frequency.

In some embodiments, said electronic circuit comprises an A/D (Analog to Digital) converter configured to convert an analog output voltage of said photodetector to digital data, and output an alert in case of deviation of said output voltage frequency from a pre-determined frequency.

In some embodiments, said electronic circuit comprises a discrete frequency detector configured to compare the frequency of an analog output voltage of said sensor to a pre-determined frequency, and output an alert in case of deviation of said frequency from said pre-determined frequency.

In some embodiments, said electronic circuit comprises a microcontroller configured to process the amount of light sensed by said photodetector and to perform computations determining one or more respiration parameters.

In some embodiments, said one or more respiration parameters comprise a respiration amplitude.

In some embodiments, said one or more respiration parameters comprise a respiration frequency.

In some embodiments, said microcontroller is further configured to compare at least one of said one or more respiration parameters to at least one pre-determined range, and to output an alert if the comparison shows a deviation above a predetermined threshold.

In some embodiments, said alert is audible.

In some embodiments, said alert is visual.

In some embodiments, said alert is tactile.

In some embodiments, the conveying of the signal related to the detected respiration comprises an alert selected from the group consisting of: an audible alert, a visual alert, a tactile alert, and an indication transmitted to a remote server.

In some embodiments, said alert is done by transmitting an indication to a remote receiver.

In some embodiments, said detector is embedded in said garment.

In some embodiments, said detector is wearable on said garment.

In some embodiments, said garment is a disposable infant diaper.

In some embodiments, said garment is a shirt.

In some embodiments, said garment is a pair of pants.

In some embodiments, said garment is an overall.

In some embodiments, said garment is an adult incontinence product.

In addition to the exemplary aspects and embodiments described above, further aspects and embodiments will become apparent by reference to the figures and by study of the following detailed description.

### BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. The figures are listed below.
Fig. 1 shows a an illustration of a diaper equipped with a light sensor, in accordance with an embodiment;
Fig. 2 shows an electronic circuit of a phototransistor sensor, in accordance with an embodiment;
Fig. 3 shows a flow chart of an exemplary algorithm for detecting breathing, in accordance with an embodiment;
Fig. 4 shows a schematic illustration of an exemplary acoustic communication interface between a sensing device and a receiving device;
Fig. 5 shows a schematic illustration of an acoustic interface of a sensing system, according to some embodiments;
Fig. 6A shows a graph of the sensors output voltage vs. time while the sensors are lying in rest on a lab table in experiment no. 1, in accordance with an embodiment;
Fig. 6B shows a graph of the sensors output voltage vs. time on the diaper within a first configuration in experiment no. 1, in accordance with an embodiment;
Fig. 6C shows a graph of the sensors output voltage vs. time on the diaper within a second configuration in experiment no. 1, in accordance with an embodiment;
Fig. 7 which shows a graph of the sensor output voltage vs. time in experiment no. 2, in accordance with an embodiment;
Fig. 8A shows a graph of the sensor output voltage vs. time on the diaper within a first configuration in experiment no. 3, in accordance with an embodiment;
Fig. 8B shows a graph of the sensor output voltage vs. time on the diaper within a second configuration in experiment no. 3, in accordance with an embodiment;
Fig. 8C shows a graph of the sensor output voltage vs. time on the diaper within a third configuration in experiment no. 3, in accordance with an embodiment;
Fig. 9A shows a graph of the sensor output voltage vs. time on the diaper within a first configuration in experiment no. 5, in accordance with an embodiment;
Fig. 9B shows a graph of the sensor output voltage vs. time on the diaper within a second configuration in experiment no. 5, in accordance with an embodiment;
Fig. 9C shows a graph of the sensor output voltage vs. time on the diaper within a third configuration in experiment no. 5, in accordance with an embodiment; and
Fig. 9D shows a graph of the sensor output amplitude vs. frequency, in accordance with an embodiment.

### DETAILED DESCRIPTION

Disclosed herein are a device and a system for sensing and monitoring respiration. One or multiple sensors may be attached to, wear on, embedded in or integrally formed with a wearable garment, such as a disposable infant diaper, pants, shirt, overall, adult incontinence product, etc. in order to sense respiration of the wearer. Furthermore, in some embodiments, sensing of the respiration is performed through a plurality of garment layers, for example through a diaper and pants worn over the diaper. Advantageously, the present sensor(s) detect respiration using optical means.

The sensing of respiration may be advantageous, in particular, for un-weaned infants as well as for certain individuals under medical attention, such as elderly patients. For example, parents of an un-weaned infant may desire to be able to monitor such parameters and be alerted when a change occurs.

The sensor may output an alert, such as an audio, visual and/or tactile alert, when an irregular change in respiration may be detected. The alert may be aimed at the parents and/or the infant itself. In an embodiment, a photodetector (also "light sensor") such as CCD, CMOS, LDR (Light Dependent Resistor), photovoltaic cell, photodiodes (operative in photovoltaic mode or photoconductive mode), phototransistor, etc. may be used for detecting respiration, based on the principle of a varying output signal dependant on reflected light which varies due to breathing movements.

The sensor may output an alert, such as an audio, visual and/or tactile alert, when an irregular change in respiration may be detected. The alert may be aimed at the parents and/or the infant itself. In an embodiment, a photodetector (also "light sensor") such as CCD, CMOS, LDR (Light Dependent Resistor), photovoltaic cell, photodiodes (operative in photovoltaic mode or photoconductive mode), phototransistor, etc. may be used for detecting respiration, based on the principle of a varying output signal dependant on reflected light which varies due to breathing movements.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

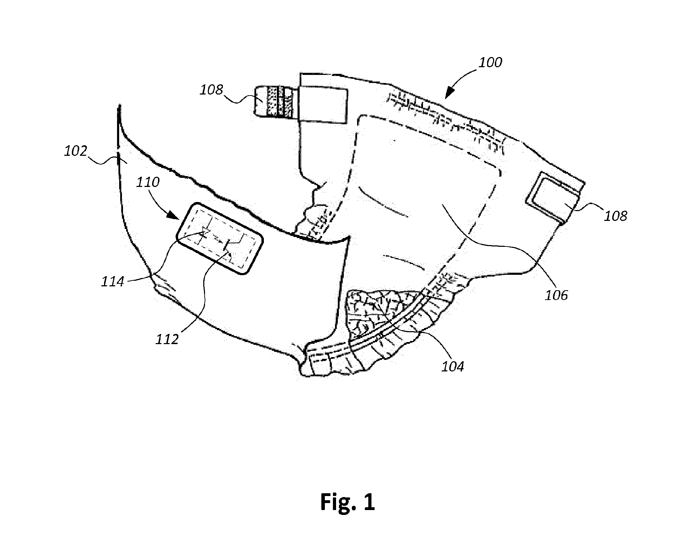

The present disclosure may be better understood with reference to the accompanying figures. Reference is now made to Fig. 1, which shows an illustration of a diaper equipped with a light sensor. The basic layers found in many modern diapers, such as diaper **100,** are commonly: (a) an outer shell **102,** commonly made of a breathable polyethylene film or a nonwoven and film composite, which prevents wetness and soil transfer to the outside environment; (b) an inner absorbent layer **104,** usually containing a mixture of air-laid paper and superabsorbent polymers; and (c) a layer **106** nearest the skin, oftentimes made of a nonwoven material with a distribution layer directly beneath, which transfers wetness to the absorbent layer. A pair of fasteners **108** is commonly used to close the diaper around the wearer's abdomen. In an embodiment, diaper **100** may embed on outer shell **102** a respiration sensor **110.** Sensor **110** may be also wearable on diaper **100** by a Velcro stripe, a multi-use adhesive patch, etc. Sensor **110** may include a photodetector **112,** an illuminator such as a LED (Light Emitting Diode) **114** and an electronic circuit (not shown here). Sensor **110** may be positioned on the front top outer margins of the diaper, which covers the infant's lower abdomen. In addition, components of sensor **110** may be distributed in different layers and/or in different positions. LED **114** may be configured to illuminate upon the diaper fabric, while photodetector **112** may be configured to receive the light reflections from diaper fabric. While the infant is breathing, the diaper fabric may be stretched and loosened, and/or the distance between his or her stomach to the diaper may decrease and increase in the frequency of breathing. As a result, the light amount reflected to photodetector **112** may also fluctuate in the frequency of breathing.

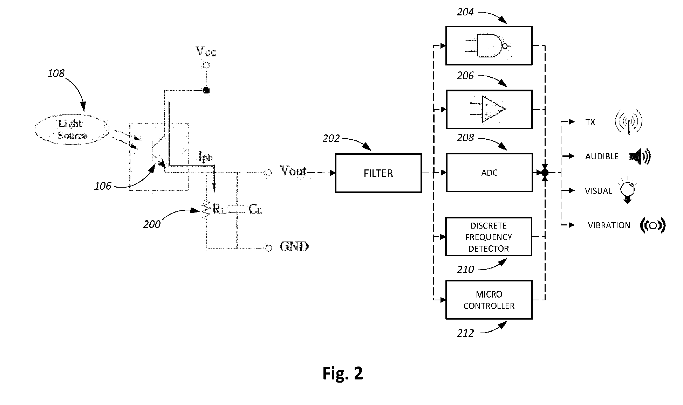

Reference is now made to Fig. 2, which shows an electronic circuit of an exemplary phototransistor-based sensor. When there is no reflection of light from light source **108,** the current through phototransistor **106** (also referred as Iₚₕ) may be approximately zero. When there is a reflection of light from light source **108,** Iₚₕ may increase, forming a voltage on a load resistor **200** (also referred as R_{L}). This may yield *V_{oUT} = Iₚₕ * R_{L}.* The output signal may then be filtered by a filter **202** to a desired frequency, and then may be amplified and serve as an input to a variety of implementations, such as a logic gate **204,** a threshold comparator **206,** an ADC (Analog to Digital) converter **208,** a discrete frequency detector **210** which may determine if an alert should be issued depending on the preset of the circuit, etc. In another embodiment, the data may be fed to a microcontroller **210** (e.g. P/N PIC18F46J50 made by Microchip) for more complex processing. Microcontroller **210** may compute respiration parameters such as amplitude and/or frequency, for example by FFT (Fast Fourier Transform), compare them to pre-determined ranges, and in case of deviation, trigger an output.

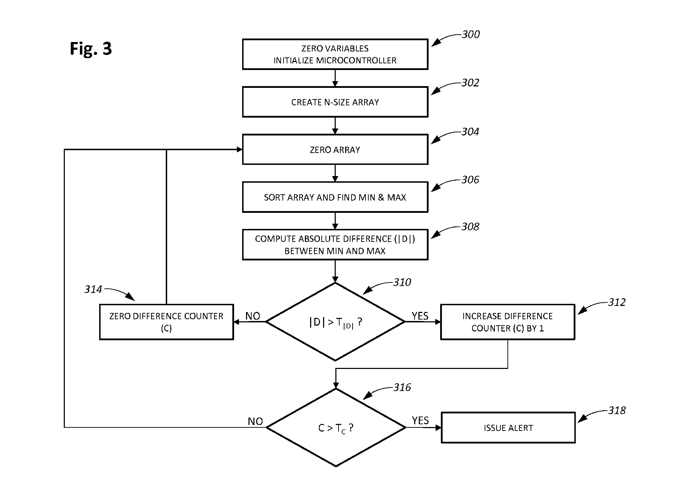

Microcontroller **210** may perform another exemplary algorithm for detecting breathing. Reference is now made to Fig. 3, which shows a flow chart of an exemplary algorithm for detecting breathing. Initially, variables may be zeroed and the microcontroller may be initialized **300.** An *N*-sized array may be then created **302,** and used for storing the last *N* consecutive sensor readings. The *N*-sized array may then be zeroed **304.** *N* may include a number of cells corresponding, for examples, to a few seconds or a few dozen seconds, based on the sampling rate of the sensor. Minimum and maximum values may then be identified in the array **306,** and the difference between them may be computed **308.** If the absolute value of the difference |*D*| may be larger than a pre-set threshold *T*_{|*D*|} **310,** then a difference counter C may be increased by one **312.** If not, the difference counter C may be zeroed **314,** and the algorithm may zero the array **304** and may continue to receive sensor readings, although alternatively, the array may not be zeroed but simply continue to receive values, in a "sliding window" configuration. Each time after the difference counter C may be increased by one **312,** the algorithm may check if the value of C may have exceeded a pre-set threshold *T_{C}* **316,** namely - has |*D*| exceeded *T*_{|*D*|} more than *T_{C}* times. If it did not, the algorithm may return to zero the array **304** (or working in a sliding window mode, as discussed above). *T*_{C} may be set to a value balancing between false positives and false negatives. When *T_{C}* may be exceeded, an alert may be issued **318.**

For any implementation, the output may be in a form of a vibration (tactile) alert, and/or visual alert, and/or vocal alert, and/or transmit alert indication to a distant receiver (not shown here).

According to some embodiments, the sensor disclosed herein may further interface and/or communicate with an external and/or remote device to convey a respiration-related signal generated by the sensor(s) disclosed herein to the device (herein, a "receiver" or a "receiving device"). Conveying the signal from the sensor of the sensing device to the receiving device may be performed by various communication routes, such as radio frequency (e.g. BlueTooth, Wifi, etc.) or acoustic communication. Acoustic communication makes use of sound and/or ultrasound, whereby a "transmitter" produces a sound that is detected by a "receiver". Sound is produced by the transmitter when a physical object vibrates rapidly, disturbs nearby air molecules (or other surrounding medium) and generates compression waves that travel in all directions away from the source. Sound can be made to vary in frequency (high pitch vs. low pitch), amplitude (loudness), and periodicity (the temporal pattern of frequency and amplitude). Since acoustic waves move rapidly through the medium, acoustic signals can be quickly started, stopped, or modified to send a time-sensitive message.

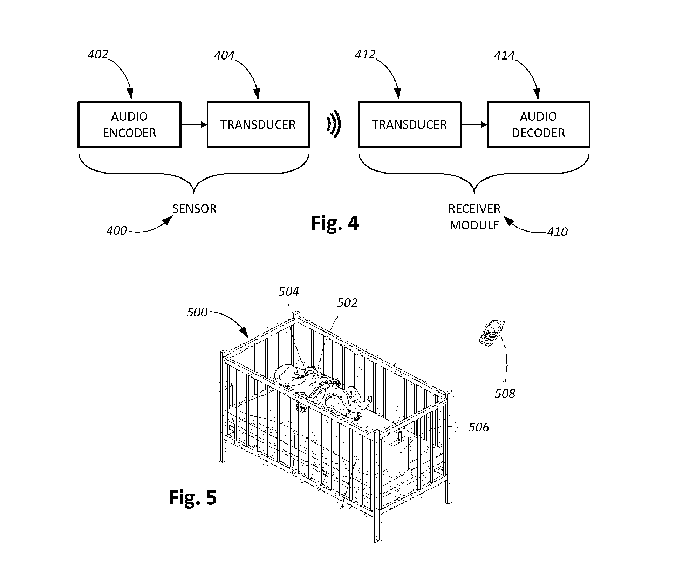

Reference is now made to Fig. 4, which shows a schematic illustration of an exemplary acoustic communication interface between a sensing device and a receiving device, together forming a respiration monitoring system.

Sensing device **400** includes an audio encoder **402,** adapted to produce an electrical signal based on the respiration-related signal produced by the sensor (namely, by the electronic circuit of the sensor). Audio encoder **402** may be incorporated in the microcontroller discussed earlier, or be connected to it. The sensing device further includes a transducing element **404,** adapted to convert the electrical signal received from audio encoder **402** into an acoustic signal transmitted towards the remote receiver. In some exemplary embodiments, the transducing element **404** is a speaker. The acoustic signal produced by the sensing device may then be received by transducer unit **412** of receiving device **410** and be converted into an electrical signal. In some exemplary embodiments, transducer **412** is a microphone. The electrical signal may then be decoded by audio decoder **414** (e.g., being part of an electronic circuit) of the receiving device. Decoding the electrical signal may be used to extract meaningful information pertaining to respiration from the signal. The decoded signal may be processed and conveyed to a user. In some embodiments, the decoded signal may be converted to an alarm signal that may a visual signal, a tactile signal, an audible signal, and the like, or any combination thereof.

According to some embodiments, the receiving device may be portable. In some embodiments, the receiving device may be placed in the vicinity of the sensing device. In some embodiments, the receiving device may be placed at a remote location, but still in acoustic communication range from the transmitting device. In some exemplary embodiments, the receiving device is a smart phone. In some exemplary embodiments, the receiving device is configured to communicate with a smart phone.

In an alternative embodiment (now shown), each of the sensing device and the receiving device may be equipped with a wireless radio module, such as a BlueTooth module, a WiFi (Wireless Fidelity) module, etc., and the communication between these two devices may be carried out using radio signals instead of using acoustic ones.

Reference is now made to Fig. 5, which shows a schematic illustration of an acoustic interface of a respiration monitoring system, according to some embodiments. In a system **500,** a sensing device **502** is placed on a subject (exemplary baby **504**). When an event is detected by the sensor of the sensing device, an acoustic alert is produced by the sensing device. The acoustic alert is detected by a receiving device such as receiving device **506,** which is located in the proximity of the subject. The receiving device may then issue an alert (such as audible, tactile and/or visual alert) to a supervisor. Additionally or alternatively, the receiving device may serve as a relay station configured to communicate with a remote computerized device (such as smart phone **508**), which is, in turn, configured to generate an appropriate alarm to the supervisor. In another embodiment, remote device **508** may embed receiving device **506,** and no relaying device may be required.

In some embodiments, the receiving device is configured to communicate with the remote device via the Internet and/or via short-range radio, utilizing technologies such as WiFi, Bluetooth, SMS, cellular data communication, push notification protocol, and activate the alarm therein, in order to notify a supervisor which may be located in a remote location. The remote device may execute an application for communicating with the receiving device and to produce audible and/or visual alarm and/or tactile alarms.

### EXPERIMENTAL RESULTS

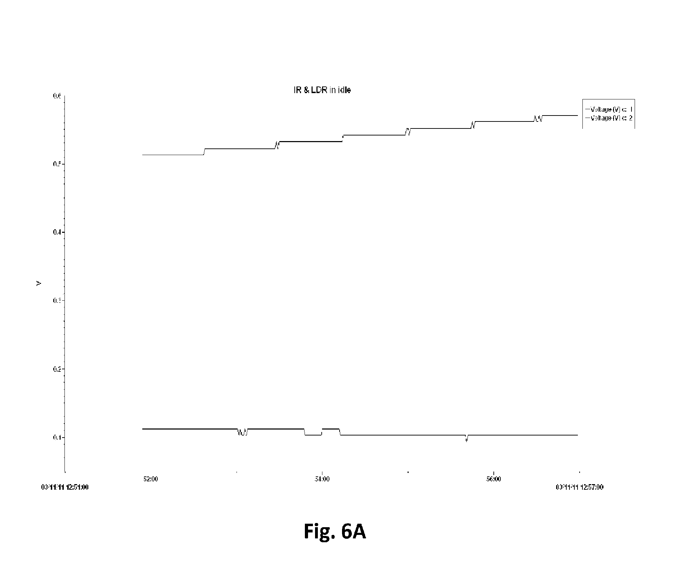

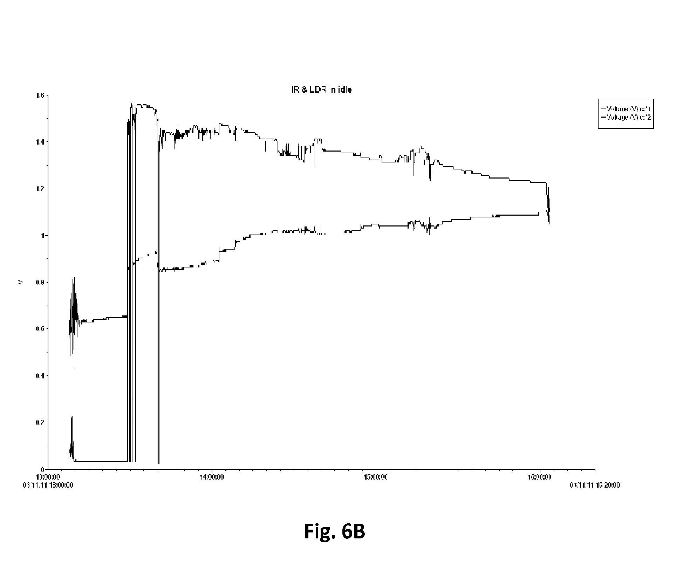

Multiple experiments have been conducted in order to establish the efficacy of present embodiments, and show their supremacy over other configurations. In a first experiment (also referred as experiment no. 1), an LDR (Light Dependent Resistor) (P/N PDV-P5001-ND made by Advanced Photonix) and an IR phototransistor (P/N QRE1113GR made by Fairchild) have been used, side by side. Reference is now made to Fig. 6A, which shows a graph of the sensors output voltage vs. time while the sensors are lying in rest on a lab table in experiment no. 1. As one can see, there is hardly a change in the output voltage, as expected. Within a first configuration, the sensors then have been positioned on the front top part of a diaper, and the LDR has been illuminated with a red LED. The results can be seen in Fig. 6B, which shows a graph of the sensors output voltage vs. time on the diaper within a first configuration in experiment no. 1. As one can see, the output voltage does not reflect normal breathing.

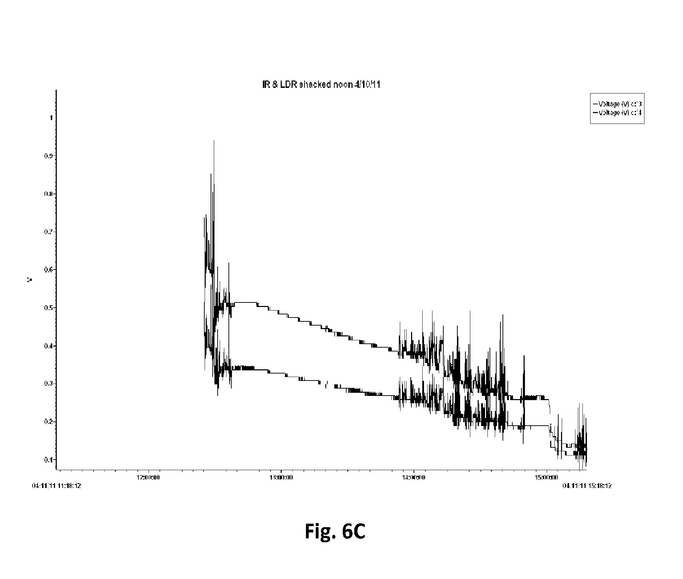

The sensors then have been re-configured to a second configuration: the IR phototransistor has been illuminated with a red LED, and voltage divider resistors have been modified. The results can be seen in Fig. 6C, which shows a graph of the sensors output voltage vs. time on the diaper within a second configuration in experiment no. 1. As one can see, the sensors are more responsive, but still the output voltage doesn't reflect the breathing.

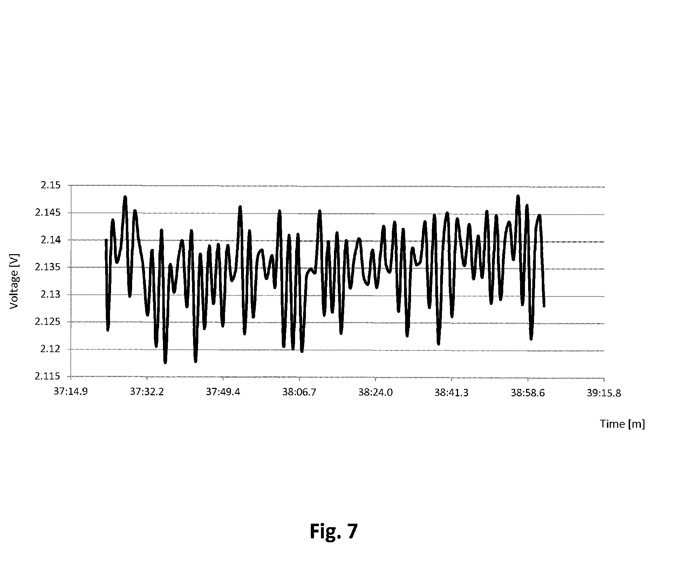

In a second experiment (also referred as experiment no. 2), a photodiode for 630nm wavelength (P/N ALS-PT19-315C/L177/TR8 made by Everlight) has been used. The photodiode has been positioned on the front top part of a diaper and illuminated with a red LED. The results can be seen in Fig. 7, which shows a graph of the sensor output voltage vs. time in experiment no. 2. As one can see, the output voltage oscillations reflect the breathing frequency in a good manner, and there is a variance of 10-30mV in the amplitude.

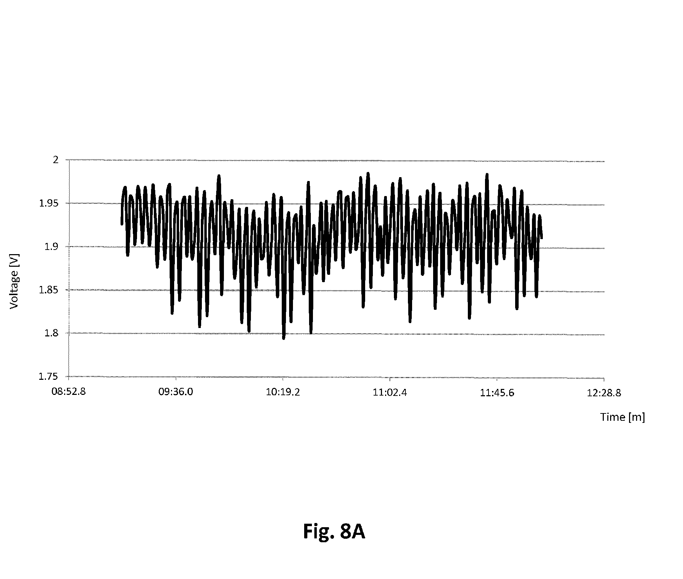

In a third experiment (also referred as experiment no. 3), a photodiode for a 620nm wavelength (P/N APDS-9004-0 made by Avago) has been used. Within a first configuration, the sensor has been positioned on the front top part of a diaper, and the photodiode has been illuminated with a white LED. The results can be seen in Fig. 8A, which shows a graph of the sensor output voltage vs. time on the diaper within a first configuration in experiment no. 3. As one can see, the output voltage oscillations reflect the breathing frequency in a good manner, and there is a variance of 30-100mV in the amplitude.

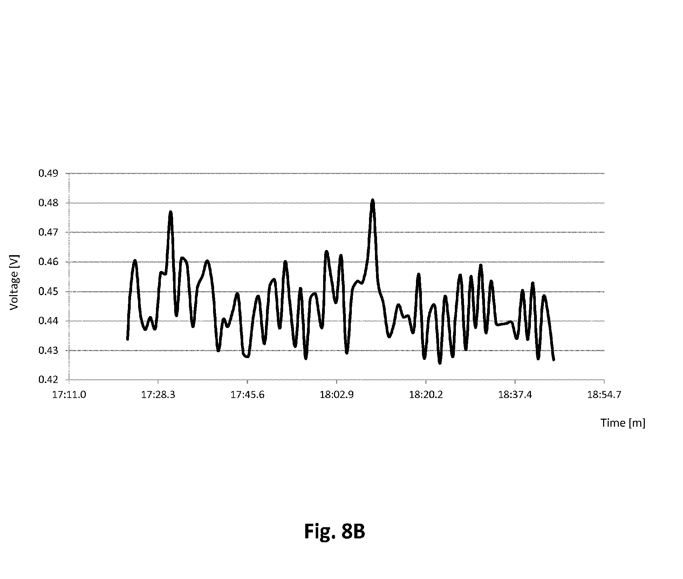

The sensor then has been re-configured to a second configuration: it was re-positioned on the front middle part of a diaper (which is lower than the position of the first configuration), while still illuminated with a white LED. The results can be seen in Fig. 8B, which shows a graph of the sensor output voltage vs. time on the diaper within a second configuration in experiment no. 3. As one can see, the output voltage oscillations reflect the breathing frequency in a good manner, and there is a variance of 20-50mV in the amplitude.

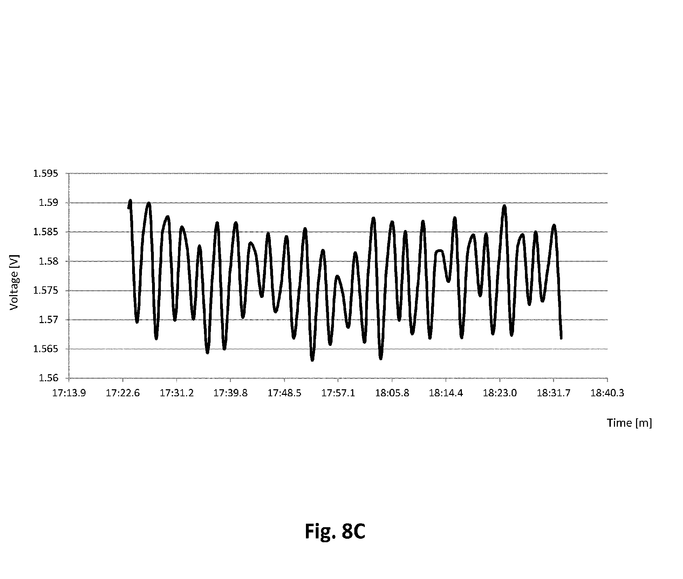

The sensor then has been re-configured to a third configuration: it was kept on the front middle part of a diaper (which is lower than the position of the first configuration), while still illuminated with a red LED. The results can be seen in Fig. 8C, which shows a graph of the sensor output voltage vs. time on the diaper within a third configuration in experiment no. 3. As one can see, the output voltage oscillations reflect the breathing frequency in a good manner, and there is a variance of 10-30mV in the amplitude. To conclude this experiment, it may be advantageous to locate the sensor on the front top part of a diaper, and to illuminate it by a white LED.

In a fourth experiment (also referred as experiment no. 4), a photodiode for 565nm (P/N TEMT6200FX01 made by Avago) has been used. The photodiode has been positioned on the front middle part of a shirt and illuminated with a red LED. The output voltage oscillations reflected the breathing frequency in a good manner, and there was a variance of 10-50mV in the amplitude. The sensor has been re-positioned on the front top part of a diaper and illuminated with a red LED (experiment no. 2 configuration), and, as expected, yielded very similar results to these of experiment no. 2.

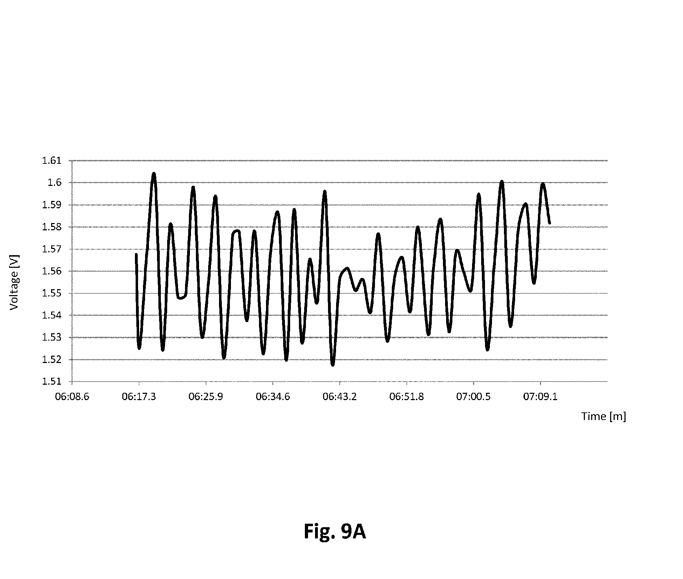

In a fifth experiment (also referred as experiment no. 5), a 635nm wavelength phototransistor and an IR phototransistor have been used. Within a first configuration, the 635nm wavelength phototransistor sensor has been positioned on the front top part of a diaper, and illuminated with a red LED. The results can be seen in Fig. 9A, which shows a graph of the sensor output voltage vs. time on the diaper within a first configuration in experiment no. 5. As one can see, the output voltage oscillations reflect the breathing frequency in a good manner, and there is a variance of 30-100mV in the amplitude.

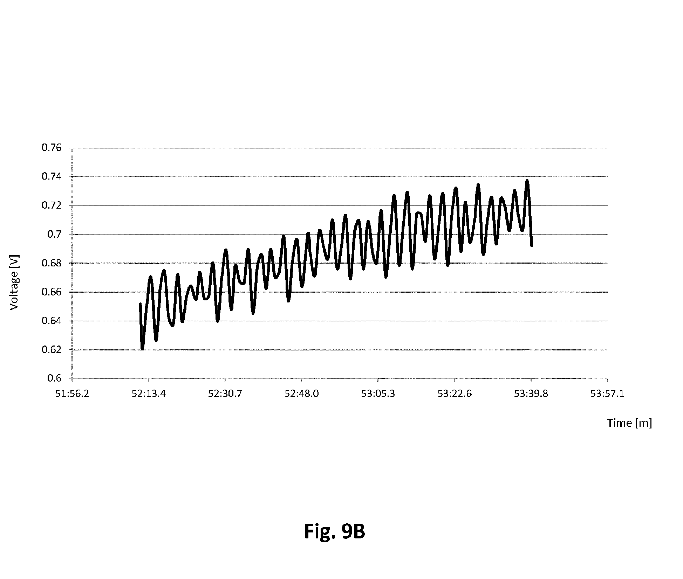

Within a second configuration, the IR phototransistor sensor has been positioned on the front top part of a diaper. The results can be seen in Fig. 9B, which shows a graph of the sensor output voltage vs. time on the diaper within a second configuration in experiment no. 5. As one can see, the output voltage oscillations reflect the breathing frequency in a good manner, and there is a variance of 20-50mV in the amplitude.

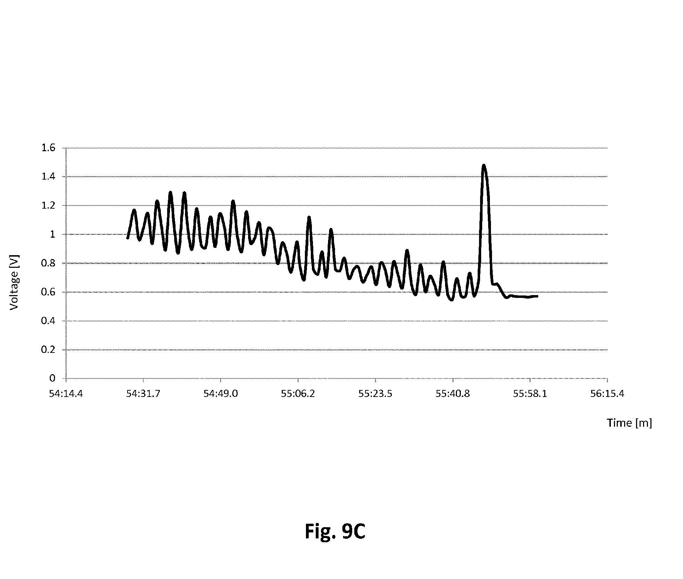

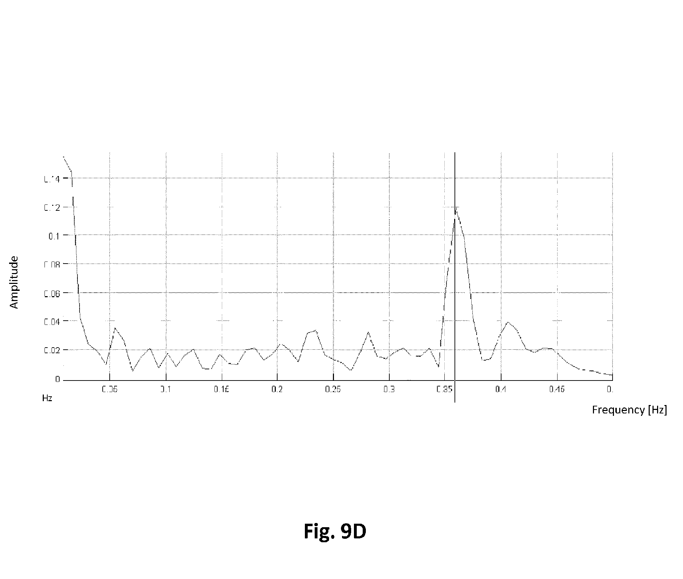

Within a third configuration, the IR phototransistor sensor has been positioned on the front most top part of a diaper (which is higher than the position of the second configuration). The results can be seen in Fig. 9C, which shows a graph of the sensor output voltage vs. time on the diaper within a third configuration in experiment no. 5. As one can see, the output voltage oscillations reflect the breathing frequency in a good manner, and there is a variance of 100-300mV in the amplitude. An FFT conversion to the frequency domain was done. The results can be seen in Fig. 9D, which shows a graph of the sensor output amplitude vs. frequency. As one can see, there is an explicit peak of energy at about 0.36Hz, which is the breathing frequency.

In the description and claims of the application, each of the words "comprise" "include" and "have", and forms thereof, are not necessarily limited to members in a list with which the words may be associated. In addition, where there are inconsistencies between this application and any document incorporated by reference, it is hereby intended that the present application controls.

## Claims

1. A garment-mountable respiration sensor, comprising:
a light source configured to illuminate said garment;
a photodetector configured to sense an amount of light reflected from said garment; and
an electronic circuit configured to detect respiration based on a temporal pattern of the amount of light sensed by said photodetector.

2. The sensor according to claim 1, wherein:
said light source is a white LED (Light Emitting Diode); and
said sensor comprises a phototransistor configured to output a signal in relation to the amount of light sensed, wherein said phototransistor is configured to sense light in the visible light range.

3. The sensor according to claim 1, wherein:
said light source is a red LED (Light Emitting Diode); and
said sensor comprises a phototransistor configured to output a signal in relation to the amount of light sensed, wherein said phototransistor is configured to sense light in the IR (Infra Red) range.

4. The sensor according to claim 1, wherein said electronic circuit comprises a logic gate configured to convert an analog output voltage of said photodetector to digital data, and output an alert in case of deviation of said output voltage frequency from a pre-determined frequency.

5. The sensor according to claim 1, wherein said electronic circuit comprises a threshold comparator configured to convert an analog output voltage of said photodetector to digital data, and output an alert in case of deviation of said output voltage frequency from a pre-determined frequency.

6. The sensor according to claim 1, wherein said electronic circuit comprises an A/D (Analog to Digital) converter configured to convert an analog output voltage of said photodetector to digital data, and output an alert in case of deviation of said output voltage frequency from a pre-determined frequency.

7. The sensor according to claim 1, wherein said electronic circuit comprises a discrete frequency detector configured to compare the frequency of an analog output voltage of said sensor to a pre-determined frequency, and output an alert in case of deviation of said frequency from said pre-determined frequency.

8. The sensor according to claim 1, wherein said electronic circuit comprises a microcontroller configured to process the amount of light sensed by said photodetector and to perform computations determining one or more respiration parameters,
wherein said one or more respiration parameters are selected from the group consisting of: a respiration amplitude and a respiration frequency, and
wherein said microcontroller is further configured to compare at least one of said one or more respiration parameters to at least one pre-determined range, and to output an alert if the comparison shows a deviation above a predetermined threshold.

9. The sensor according to claim 8, wherein said alert is selected from the group consisting of: an audible alert, a visual alert, a tactile alert, and an indication transmitted to a remote server.

10. The sensor according to claim 1, wherein:
said detector is embedded in said garment; or
said detector is wearable on said garment.

11. The sensor according to claim 1, wherein said garment is selected from the group consisting of: a disposable infant diaper, a shirt, a pair of paints, an overall and an adult incontinence product.

12. A respiration monitoring system comprising:
a garment-mountable respiration sensor comprising:
(a) a light source configured to illuminate said garment,
(b) a photodetector configured to sense an amount of light reflected from said garment,
(c) a transducing element,
(d) an electronic circuit configured to detect respiration based on a temporal pattern of the amount of light sensed by said photodetector, wherein said electronic circuit further comprises an audio encoder configured to encode a signal related to the detected respiration using said transducing element, and to cause said transducing element to transmit the encoded signal as an acoustic signal; and
a receiving device configured to receive and decode the acoustic signal, and to convey the signal related to the detected respiration to a user.

13. The respiration monitoring system according to claim 12, wherein:
said light source is a white LED (Light Emitting Diode); and
said sensor comprises a phototransistor configured to output a signal in relation to the amount of light sensed, wherein said phototransistor is configured to sense light in the visible light range.

14. The respiration monitoring system according to claim 12, wherein:
said light source is a red LED (Light Emitting Diode); and
said sensor comprises a phototransistor configured to output a signal in relation to the amount of light sensed, wherein said phototransistor is configured to sense light in the IR (Infra Red) range.

15. The respiration monitoring system according to claim 12, wherein the conveying of the signal related to the detected respiration comprises an alert selected from the group consisting of: an audible alert, a visual alert, a tactile alert, and an indication transmitted to a remote server.
